(19) Europäisches Patentamt
European Patent Office
Office européen des brevets

(11) Veröffentlichungsnummer : **0 034 364**
**B1**

(12) # EUROPÄISCHE PATENTSCHRIFT

(45) Veröffentlichungstag der Patentschrift : **07.11.84**

(51) Int. Cl.³ : **C 07 J 41/00**, C 07 J 9/00

(21) Anmeldenummer : **81101114.7**

(22) Anmeldetag : **17.02.81**

(54) **Neue 20-substituierte 3-Oxo-pregna-4-en-steroidverbindungen und Verfahren zu ihrer Herstellung.**

(30) Priorität : **19.02.80 US 122397**

(43) Veröffentlichungstag der Anmeldung : **26.08.81 Patentblatt 81/34**

(45) Bekanntmachung des Hinweises auf die Patenterteilung : **07.11.84 Patentblatt 84/45**

(84) Benannte Vertragsstaaten : **AT BE CH DE FR GB IT LI LU NL SE**

(56) Entgegenhaltungen :
**US-A- 4 088 760**
**JOURNAL OF THE CHEMICAL SOCIETY Part IV, November 1969, Seiten 3194-3202 London, G.B. A.F. CHAPLIN et al.: "The steroid series. Part IV. Some basic derivatives"**

(73) Patentinhaber : **HENKEL CORPORATION**
**7900 West 78th Street**
**Minneapolis Minnesota 55435 (US)**

(72) Erfinder : **Krbechek, Leroy O.**
**2041 Orkla Drive**
**Minneapolis Minnesota 55427 (US)**

(74) Vertreter : **Fues, Johann Friedrich, Dr. et al**
**Deichmannhaus am Hauptbahnhof**
**D-5000 Köln 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 034 364 B1

**Beschreibung**

Die Erfindung betrifft neue 20-substituierte Steroidverbindungen, die bedeutungsvolle Derivate mit Steroidgrundstruktur und insbesondere wichtige Zwischenprodukte bei der Synthese von Progesteron und progesteronartigen Verbindungen aus Steroidverbindungen, beispielsweise pflanzlichen oder tierischen Ursprungs, durch Abbau des Seitenketten-Substituenten in C-17 des Steroidgerüsts sind. Weiterhin betrifft die Erfindung das Verfahren zur Herstellung dieser neuen 20-substituierten Steroidderivate.

Gegenstand der offengelegten Europäischen Patentanmeldung 004913 ist u. a. ein Verfahren zur Herstellung von 3-Oxo-pregna-4-en-20-carbonsäure ($\Delta^4$-BNC) und/oder 3-Oxo-pregna-1,4-dien-20-carbonsäure ($\Delta^{1,4}$-BNC) durch mikrobiellen Seitenkettenabbau an 17-C-Seitenketten-Steroidsubstraten. Besonders geeignete Ausgangsmaterialien sind Sterine tierischen und/oder pflanzlichen Ursprungs, wie Cholesterin, Sitosterin, Stigmasterin und verwandte Verbindungen. $\Delta^4$- und $\Delta^{1,4}$-BNC sind hierdurch wohlfeile, technisch zugängliche Ausgangsmaterialien für die Gewinnung von Progesteron und verwandten Verbindungen geworden. Die Europäische Patentanmeldung EP-A-81 100 146.0 (33439) beschreibt eine Modifikation des Verfahrens, durch die 3-Oxo-pregna-1,4,17(20)-carbonsäure ($\Delta^{1,4,17(20)}$-BNC) in entsprechender Weise zugänglich geworden ist. Die Herstellung von 3-Oxopregna-4,17(20)-dien-20-carbonsäure ($\Delta^{4,17(20)}$-BNC) durch mikrobiellen Seitenkettenabbau an Steroidsubstraten wird in der US-PS 3 994 933 geschildert.

Den hier genannten BNC-Verbindungen ist die als Substituent in 20-Stellung vorliegende Carboxylgruppe —COOH gemeinsam, die auf dem Wege zum Progesteron abgebaut werden muß, wobei weiterhin die 20-Stellung zur Oxogruppe umzuwandeln ist.

Die Erfindung geht von der Aufgabe aus, einen wesentlichen Beitrag zur Lösung dieser Problemstellung zu liefern. In der technischen Lösung dieser erfindungsgemäßen Aufgabe werden neue 20-substituierte Steroidverbindungen und das Verfahren zu ihrer Herstellung beschrieben, mittels deren ein technisch bequem gangbarer Weg zum Progesteron bzw. zu progesteronartigen Verbindungen geöffnet wird.

Gegenstand der Erfindung sind dementsprechend in einer ersten Ausführungsform neue 20-substituierte 3-Oxo-pregna-4-en- sowie ggf. wenigstens eine weitere Doppelbindung in 1 (2)- und/oder 17 (20)-Stellung aufweisende Steroidverbindungen der allgemeinen Formel I

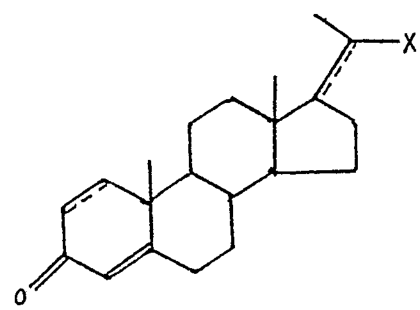

(I)

in der X die Gruppe —$CONH_2$, —NCO oder —NHCOOR bedeutet, worin R ein niederer Kohlenwasserstoffrest ist, mit Ausnahme von 3-Oxopregna-4-en-20-carboxamid.

Gegenstand der Erfindung ist in einer weiteren Ausführungsform das Verfahren zur Herstellung von 20-substituierten 3-Oxo-pregna-4-en- sowie ggf. wenigstens eine weitere Doppelbindung in 1(2)- und/oder 17(20)-Stellung aufweisenden Steroidverbindungen der zuvor angegebenen allgemeinen Formel I, wobei dieses Verfahren dadurch gekennzeichnet ist, daß man von den der allgemeinen Formel I entsprechenden 20-Carbonsäuren (X = —COOH) ausgehend zunächst das 20-Carbonsäurehalogenid (X = —CO—Halogen) herstellt und dieses mit Ammoniak oder Ammoniak liefernden Verbindungen zum 20-Carbonsäureamid (X = —$CONH_2$) umsetzt, gewünschtenfalls dieses durch Umsetzung mit Bleitetraacetat in das 20-Isocyanat (X = —NCO) umwandelt und gewünschtenfalls durch Anlagerung eines Alkohols ROH (worin R die angegebene Bedeutung hat) das 20-Carbamat (X = —NHCOOR) gewinnt.

Die vier durch die Erfindung betroffenen Grundtypen an Steroidverbindungen sind in den folgenden Formelbildern A bis D aufgezeigt.

A

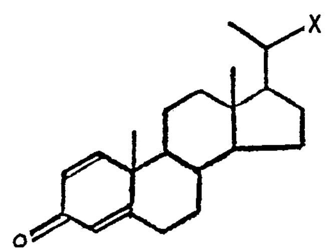

B

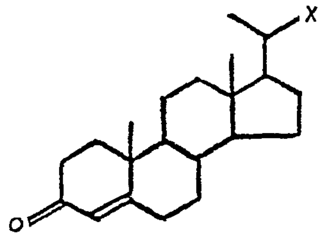

C

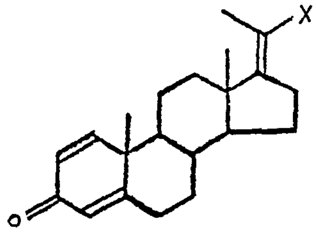

D

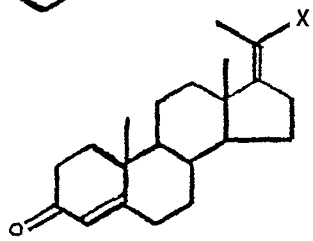

Bedeutet X die Carboxylgruppe —COOH, dann stellt A die $\Delta^{1,4}$-BNC dar, B ist die $\Delta^{4}$-BNC, C ist die $\Delta^{1,4,17}$-BNC und D ist die $\Delta^{4,17}$-BNC.

Als neue Verbindungen der Erfindung zu diesen vier Grundstrukturen werden zunächst die entsprechenden 20-Carbonsäureamide geschildert, d. h. die Verbindungen, in denen X den Rest —$CONH_2$ bedeutet. Neue Verbindungen im Sinne der Erfindung sind weiterhin die entsprechenden 20-Isocyanate, d. h. die Verbindungen gemäß A bis D, bei denen X der Rest —NCO ist. Ebenfalls neue Verbindungen im Sinne der Erfindung sind schließlich die 20-Carbamate, bei denen in den Formelbildern A bis D der Rest X die Bedeutung —NHCOOR hat, worin R eine niederer Kohlenwasserstoffrest ist. Als niederer Kohlenwasserstoffrest kommen insbesondere solche mit bis zu 10, vorzugsweise mit bis zu 5 und insbesondere mit bis zu 4 C-Atomen in Betracht. Dem Methylrest und dem tert.-Butylrest können besondere Bedeutung zukommen.

Besonders bevorzugte Steroidderivate im Sinne der Erfindung können die $\Delta^{1,4}$-Verbindungen gemäß A und die $\Delta^{1,4,17(20)}$-Verbindungen gemäß C sowie ggf. auch noch die $\Delta^{4,17(20)}$-Verbindungen gemäß D sein.

Alle hier dargestellten Steroidverbindungen der Erfindung sind durch das eingangs erwähnte Verfahren bequem zugänglich und lassen sich letzlich zu den 3,20-Dioxo-Steroidverbindungen vom Progesteron-Typ umwandeln, wie noch im einzelnen dargestellt wird.

Das erfindungsgemäße Herstellungsverfahren geht von den Steroid-20-Carbonsäuren gemäß Formelbildern A bis D aus, und überführt diese in einer ersten Stufe in die Carbonsäurehalogenide, die dann ihrerseits zu den 20-Carboxamiden umgewandelt werden. Die Umwandlung solcher Carbonsäuren in die Carbonsäurehalogenide, insbesondere in die entsprechenden Chloride, wird in der älteren Europäischen Patentanmeldung EP-A-81 100 145.2 (34248) beschrieben und beansprucht. Im einzelnen gilt das Folgende :

Eine oder mehrere der genannten Säuren werden in Form der freien Säure als Salz oder als hydrolysierbarer Ester mit einem anorganischen Halogenierungsmittel behandelt. Besonders geeignete Halogenierungsmittel sind Thionylchlorid und Thionylbromid, aber auch andere bekannte Halogenierungsmittel, wie Phosphortrichlorid, Phosphorpentachlorid, Phosphoroxychlorid, und die entsprechenden Bromide können ggf. Verwendung finden. Bevorzugt wird ein Chlorierungsmittel und insbesondere Thionylchlorid eingesetzt. Bevorzugt wird die Chlorierung bei niederen Temperaturen, insbesondere im Bereich von etwa − 20 °C bis + 20 °C, vorzugsweise bei Temperaturen nicht über 15 °C, durchgeführt. Das Chlorierungsmittel wird in wenigstens äquivalenter Menge zur vorgelegten Säure eingesetzt. Zur Vermeidung von unerwünschten Nebenreaktionen, die insbesondere bei Steroidverbindungen mit mehr als nur einer Doppelbindung im System auftreten können, kann es zweckmäßig sein, mit einem nur sehr begrenzten Überschuß des Halogenierungsmittels zu arbeiten, der beispielsweise nicht mehr als 20 %, vorzugsweise nicht mehr als 10 %, der stöchiometrisch benötigten Menge ausmacht. Geeignete Reaktionsbedingungen vorausgesetzt, kann allerdings auch mit größeren Mengen, z. B. im Bereich von 1,1 bis 5,0 Äquivalenten, insbesondere bis zu 3,0 Äquivalenten, des Halogenierungsmittels gearbeitet werden. Die Halogenierung kann in Gegenwart von Lösungsmitteln durchgeführt werden. Geeignete Lösungsmittel sind beispielsweise Halogenkohlenwasserstoffe, wie

Methylenchlorid oder Ethylendichlorid. Zweckmäßigerweise wird das Halogenierungsmittel der vorgelegten Lösung der Steroidverbindung im Lösungsmittel langsam zugesetzt. Das Halogenierungsmittel sollte so rein wie möglich sein. Es kann zweckmäßig sein, das Halogenierungsmittel vor der Verwendung einem speziellen Reinigungsschritt durch Behandlung mit ungesättigten Verbindungen, wie Leinöl oder insbesondere Squalen, zu unterwerfen. Diese ungesättigten Komponenten reagieren offenbar mit den Verunreinigungen im Halogenierungsmittel. Hierdurch wird die Bildung von unerwünschten Nebenprodukten verringert. Auch die Mitverwendung eines tertiären Amins, wie Pyridin, hat sich als hilfsreich zur Erzielung hoher Ausbeuten an reinem Säurehalogenid erwiesen.

In der nächsten Stufe des erfindungsgemäßen Verfahrens wird das Steroid-20-Carbonylhalogenid mit Ammoniak oder einer Ammoniak liefernden Verbindung zum entsprechenden Steroid-20-Carbonamid umgesetzt. Die Umsetzung mit Ammoniak bzw. Ammoniak liefernden Verbindungen wird zweckmäßigerweise im Temperaturbereich von – 20 °C bis etwa 80 °C und vorzugsweise im Bereich von – 5 °C bis etwa 35 °C durchgeführt.

Neben Ammoniak ist insbesondere Ammoniumhydroxid als Ammoniak liefernder Reaktant geeignet. Zur Bildung des Säureamids aus dem Halogenid wird wenigstens ein äquivalenter Betrag an Ammoniak benötigt, allerdings wird zur Bindung des dabei freiwerdenden Halogenwasserstoffs ein weiteres Äquivalent an basischer Komponente erforderlich. Ammoniak bzw. die Ammoniak liefernde Verbindung wird dementsprechend im Mengen bis zu beispielsweise 5 Äquivalenten, vorzugsweise in Mengen bis zu etwa 3 Äquivalenten, eingesetzt, wobei Mengen von etwa 1,2 bis 3 Äquivalenten und insbesondere von etwa 2 bis 3 Äquivalenten Ammoniak je Äquivalent Säurehalogenid besonders zweckmäßig sein können.

Die Umsetzung des Säurehalogenids mit Ammoniak bzw. der Ammoniak liefernden Verbindung wird vorzugsweise in einem organischen Lösungsmittel durchgeführt. Auch hier sind halogenierte organische Lösungsmittel, wie Halogen-Kohlenwasserstoffe und insbesondere Methylenchlorid, besonders geeignet.

Wird mit Ammoniumhydroxid als Ammoniak liefernder Verbindung gearbeitet, so enthält das Reaktionsgemisch substantielle Wassermengen. Eine einfache Phasentrennung erlaubt die Gewinnung der gewünschten Verbindung. Die organische Phase wird mehrfach mit Wasser gewaschen, vorzugsweise anschließend mit beispielsweise Calciumsulfat getrocknet und gefiltert. Das organische Lösungsmittel wird dann in bekannter Weise entfernt. Die angefallene Carboxamidoverbindung kann gewünschtenfalls durch Auswaschen mit einer organischen Komponente, wie Aceton, weitergereinigt werden, um praktisch reines Säureamid zu erhalten.

Das erfindungsgemäße Verfahren sieht in einer weiterführenden Stufe vor, gewünschtenfalls die so gewonnenen Säureamide zu den Steroid-20-Isocyanatverbindungen und ggf. zu den 20-Carbamatverbindungen umzuwandeln. Es ist dabei möglich, die Isocyanate als solche zu isolieren und sie gewünschtenfalls in einer getrennten Verfahrensstufe in die Carbamate umzuwandeln, möglich ist aber auch die Verbindung dieser beiden Verfahrensstufen zu einem in einem Zuge durchgeführten Verfahrensschritt. Im letzteren Fall wird das intermediär gebildete 20-Isocyanat in situ zum entsprechenden Carbamat umgewandelt. Im einzelnen gilt hier das Folgende :

Wird die isolierte Herstellung der Steroid-20-Isocyanate gewünscht, dann werden die Steroid-20-Carboxamide mit einer wenigstens äquivalenten Menge an Bleitetraacetat in Gegenwart eines nichtwäßrigen aprotischen Lösungsmittels umgesetzt, anschließend wird die organische Phase abgetrennt, das Lösungsmittel entfernt und die 20-Isocyanatverbindung isoliert. Die Lösungsmittel müssen hier aprotischer Natur sein, andernfalls würde das Isocyanat in unerwünschter Weise mit dem Lösungsmittel weiter reagieren. Ein geeignetes Lösungsmittel für diese Verfahrensmodifikation is beispielsweise Tetrahydrofuran.

Die Umsetzung zur Isocyanatbildung wird üblicherweise bei Temperaturen von etwa 0 bis 100 °C, vorzugsweise im Bereich von etwa 5-50 °C, durchgeführt. Bleitetraacetat ist in äquivalenter Menge zur eingesetzten Steroidverbindung erforderlich und sollte bevorzugt in nur beschränktem Überschuß eingesetzt werden. Gleichwohl gilt, daß Bleitetraacetat grundsätzlich in Mengen von etwa 1,1 bis 5 Äquivalenten, bezogen auf Steroidverbindung, Verwendung finden kann, wobei das Arbeiten mit 1,2 bis 3 Äquivalenten Bleitetraacetat bevorzugt sein kann.

Bevorzugt wird die Umsetzung der Carboxamidverbindung mit Bleitetraacetat in inerter Atmosphäre und in vollständiger Abwesenheit von Wasser durchgeführt. Hierzu wird das Reaktionsgefäß zweckmäßigerweise mehrfach mit scharf getrocknetem Stickstoff gespült, um auch Spuren von Wasser aus dem Reaktionsgefäß zu entfernen. Die Reaktion wird dann unter trockenem Stickstoff durchgeführt.

Die Isocyanat enthaltende organische Lösung kann mit Natriumthiosulfat und dann mit gesättigter Kochsalzlösung gewaschen werden, um restliche Mengen des Bleisalzes aus dem Reaktionsgemisch zu entfernen. Das gewünschte Verfahrensprodukt kann schließlich durch Entfernung des Lösungsmittels bzw. durch Lösungsmittelextraktion gewonnen werden.

In der erfindungsgemäß gewünschtenfalls durchgeführten nächsten Verfahrensstufe wird das Isocyanat mit einem einwertigen Alkohol zum Carbamat umgesetzt. Als einwertige Alkohole kommen insbesondere niedere Alkohole, insbesondere niedere Alkanole, in Betracht. Geeignet sind beispielsweise Alkanole mit bis zu 10, vorzugsweise mit bis zu 5 Kohlenstoffatomen, wobei Alkanolen mit 1 bis 4 Kohlenstoffatomen besondere Bedeutung zukommen kann. Methanol und tert.-Butanol führen zu im Rahmen der Erfindung besonders geeigneten Steroid-20-Carbamaten. Zur Gewinnung der Carbamate werden die Isocyanate in zuvor isolierter Form oder gewünschtenfalls auch ohne vorherige Isolierung mit

einer wenigstens äquivalenten Menge des Alkohols zusammengebracht. Zweckmäßigerweise wird der Alkohol im Überschuß eingesetzt. Die Reaktion erfolgt in an sich bekannter Weise und kann dementsprechend beispielsweise bei Raumtemperatur oder nur mäßig erhöhten Temperaturen vorgenommen werden. Temperaturen von 0-60 °C können geeignet sein. Der eingesetzte Alkohol ist zweckmäßigerweise vorgetrocknet und insbesondere wenigstens weitgehend wasserfrei, um unerwünschte Nebenreaktionen auszuschließen.

Die Steroid-20-Carbamate sind stabile Verbindungen, die beispielsweise durch Abziehen des Alkoholüberschusses isoliert und in konventioneller Weise gewünschtenfalls weitergereinigt werden können.

Die Steroid-20-Carbamate können anschließend hydrolytisch zu den Steroid-20-Aminen gespalten werden, die letztlich zu den Verbindungen vom Progesteron-Typ umgesetzt werden können. Einzelheiten zu diesen nachfolgenden, im Rahmen der vorliegenden Erfindung nicht mitbeanspruchten Verfahrensschritten finden sich beispielsweise in den parallelen Europäischen Patentanmeldungen EP-A-81 101 115.4 (34365) (« Verfahren zur Herstellung von 3,20-Dioxo-pregn-4-en und/oder 3,20-Dioxo-pregna-1,4-dien », US-Priorität vom 19. Februar 1980, Aktenzeichen : 122,396) EP-A-81 101 118.8 (34367) (« Verfahren zur hydrolytischen Spaltung von Steroid-20-Carbamaten », U.S. Priorität vom 19. Februar 1980, Aktenzeichen : 122,321), EP-A-81 101 113.9 (34363) (« Neue Steroidverbindung mit einer 20-Amino-Funktion und Verfahren zu ihrer Herstellung », US-Prioritäten vom 19. Februar 1980, Aktenzeichen 122 397 und 122 321).

Es sind zahlreiche chemische Transformationen an Steroidverbindungen im Stand der Technik bekanntgeworden. Gleichwohl sind die hier beanspruchten Produkte mit Steroidstruktur und das Verfahren zu ihrer Herstellung bisher nicht bekannt geworden. Aus dem umfangreichen Stand der Technik seien die folgenden Literaturstellen benannt : JACS, Band 70, No. 3 (1948), S. 887 ; US-PS 4 088 760 ; US-PS 2 566 336 ; GB-PS 1 043 018 ; US-PS 2 731 461 ; Chemische Berichte, Jahrgang 88, No. 6 (1955), S. 883-894 ; US-PS 2 752 369 ; US-PS 3 519 658 ; J. Am. Chem. Soc. 1956, S. 524 ff. ; Tetrahedron Letters No. 18, (1964) S. 1053 bis 1061 ; J. Am. Chem. Soc., Band 91 (1969), S. 1429-1432 und Band 97 (1975), S. 6900-6901 ; Helvetica Chemica Acta, Band 32, Teil V (1949), No. 233, S. 1764 ; a.a.O. Band 32, Teil V (1949), No. 232, S. 1758 ; a.a.O. Band 32, Teil VI (1949), No. 255, S. 1922.

## Beispiel

a) Herstellung der Säurechloride

3-Oxo-pregna-1,4-dien-20-carbonsäure, 3-Oxo-pregna-4-en-20-carbonsäure, 3-Oxo-pregna-1,4,17(20)-trien-20-carbonsäure und/oder 3-Oxo-pregna-4,17(20)-dien-20-carbonsäure werden durch Umsetzung mit Thionylchlorid bei 0 °C unter Verwendung eines leichten stöchiometrischen Überschusses in Methylenchlorid zu den entsprechenden Säurechloriden umgewandelt. Die Reaktion ist nach 20 Minuten im wesentlichen abgeschlossen. Das Säurechlorid wird isoliert, gereinigt und identifiziert.

Im folgenden wird die entsprechende Darstellung des $\Delta^{1,4,17}$-BNC-Chlorids beschrieben, die Herstellung der anderen Säurechloride erfolgt in entsprechender Weise.

17 g (50 mmol) $\Delta^{1,4,17}$-BNC in 100 ml absolutem Methylenchlorid werden bei 0 °C mit 4,0 ml (55 mmol) frisch über Squalen-destilliertem Thionylchlorid versetzt und 20 Minuten bei 0 °C gerührt. Danach werden das Lösungsmittel und überschüssiges Thionylchlorid bei der gleichen Temperatur im Vakuum entfernt. Der Rückstand wird wieder in Methylenchlorid aufgenommen und die Lösung nochmals zur Trockne eingehängt. Der Rückstand ist für weitere Umsetzungen zu verwenden. Um ein zur Analyse geeignetes Säurechlorid zu erhalten, wird das rohe Säurechlorid mit absolutem Äther digeriert und nach dem Abziehen des Äthers sorgfältig an der Ölpumpe getrocknet.

Elementaranalyse

| | | | |
|---|---|---|---|
| Berechnet : | C 73,2 | H 8,10 | Cl 9,82 |
| Gefunden : | C 72,85 | H 8,22 | Cl 10,1 |

b) Herstellung der Säureamide

Die gemäß a) gewonnenen Säurechloride werden mit konzentrierter wäßriger Ammoniaklösung bei 0 °C zur Umsetzung gebracht. Die Ammoniakmenge beträgt dabei wenigstens das Doppelte des stöchiometrisch benötigten Betrages, wird jedoch zweckmäßigerweise in einem darüberhinausgehenden Überschluß eingesetzt. Die Säurechloride werden in Methylenchlorid als Lösungsmittel gelöst zur Umsetzung gebracht.

Nach Abschluß der Reaktion werden die beiden Flüssigphasen voneinander getrennt. Die wäßrige Phase wird mehrfach mit Methylenchlorid ausgewaschen und jeweils die organische Phase wieder abgetrennt. Die organischen Phasen werden vereinigt, gewaschen und die Lösung zur Trockne eingeengt. Das rohe Säureamid wird durch Digerieren mit Aceton gereinigt und zur Analyse sorgfältig an der Ölpumpe getrocknet.

Analysenwerte

$\Delta^{1,4}$-BNC-Amid
Fp : 235-238 °C

Elementaranalyse :

Berechnet : C 77,38 H 9,15 N 4,10
Gefunden : C 77,4 H 9,03 N 4,05

$^1$H-NMR-Spektrum (90 MHz, $CDCl_3$, δ-Werte) :
18-$CH_3$ 0,74 ppm, s
19-$CH_3$ 1,21 ppm, s
21-$CH_3$ 1,17 ppm, d (J = 8 Hz)
— olefinische Protonen : ABC-System mit Signalen bei : 6,03 ; 6,11 ; 6,13 ; 6,22 ; 6,25 ; 7,00 ; 7,11 ppm

$\Delta^{4}$-BNC-Amid
Fp : 183-185 °C

Elementaranalyse

Berechnet : C 76,92 H 9,68 N 4,08
Gefunden : C 76,8 H 9,75 N 4,12

$^1$H-NMR-Spektrum (8 MHz, $CDCl_3$, δ-Werte)
18-$CH_3$ 0,73 ppm, s
19-$CH_3$ 1,18 ppm, s
21-$CH_3$ 1,20 ppm, d (J = 8 Hz)
— olefinisches Proton : 5,71 ppm, s (verbreitert)

$\Delta^{1,4,17}$-BNC-Amid

Elementaranalyse

Berechnet : C 77,84 H 8,61 N 4,13
Gefunden : C 77,2 H 8,43 N 3,98

$\Delta^{4,17}$-BNC-Amid

Elementaranalyse

Berechnet : C 77,38 H 9,15 N 4,10
Gefunden : C 77,1 H 8,95 N 3,93

c) Herstellung der Isocyanate

Die gemäß b) gewonnenen Säureamide werden in Tetrahydrofuran gelöst und unter Stickstoff mit einem geringen Überschuß an Bleitetraacetat zur Umsetzung gebracht. Zuvor ist das gesamte Reaktionssystem durch Stickstoffspülung gereinigt worden. Das Reaktionsgemisch wird für einen Zeitraum von 1,5 Stunden gerührt, danach ist die Reaktion abgeschlossen. Anschließend wird Natriumthiosulfat zur Reduktion eines eventuell noch vorliegenden Überschusses des Bleisalzes zugefügt. Die Reaktionslösung wird ein zweites Mal mit wäßrigem Thiosulfat und dann drei Mal mit gesättigter Kochsalzlösung gewaschen. Nach Phasentrennung und Trocknung der organischen Phase wird das Lösungsmittel abgezogen. Als Rückstand fallen die Isocyanatverbindungen an.

Analysenwerte

$\Delta^{1,4}$-BNC-Isocyanat

Elementaranalyse

Berechnet : C 77,84 H 8,61 N 4,13
Gefunden : C 77,44 H 8,45 N 3,96

$^1H$-NMR-Spektrum (90 MHz, $CDCl_3$, δ-Werte) :
18-$CH_3$ 0,73 ppm, s
19-$CH_3$ 1,20 ppm, s
21-$CH_3$ 1,28 ppm, d (J = 7 Hz)
20-CH 3,51 ppm, m
— olefinische Protonen : ABC-System mit Signalen bei : 6,02 ; 6,10 ; 6,12 ; 6,21 ; 6,23 ; 6,93 ; 7,05 ppm
IR-Spektrum ($CH_2$ $Cl_2$ [$cm^{-1}$]) : 2 270, 1 662, 1 621, 1 602

$\Delta^4$-BNC-Isocyanat

Elementaranalyse

| | | | |
|---|---|---|---|
| Berechnet : | C 77,38 | H 9,15 | N 4,10 |
| Gefunden : | C 77,0 | H 8,91 | N 3,97 |

d) Herstellung der Carbamate

Die gemäß c) isolierten Isocyanatverbindungen werden in einem Überschuß eines niederen Alkohols, beispielsweise eines C-1 bis C-4 Alkoholes, und insbesondere in Methanol oder tert.-Butanol gelöst und zur Umsetzung gebracht. Der Überschuß des Alkohols wird abgezogen. Das entstandene Carbamat wird isoliert.

Analysenwerte

$\Delta^{1,4}$-BNC-Propylcarbamat
Fp 159-161 °C

Elementaranalyse

| | | | |
|---|---|---|---|
| Berechnet : | C 75,15 | H 9,33 | N 3,51 |
| Gefunden : | C 74,9 | H 9,20 | N 3,42 |

$^1H$-NMR-Spektrum (90 MHz, $CDCl_3$, δ-Werte) :
18-$CH_3$ 0,77 ppm, s
O—C—C—$CH_3$ 0,90 ppm, t (J = 7,5 Hz)
21-$CH_3$ 1,13 ppm, d (J = 7 Hz)
19-$CH_3$ 1,20 ppm, s
20-CH 3,65 ppm, m
O—$CH_2$— 3,97 ppm, t (J = 7 Hz)
— olefinische Protonen : ABC-System mit Signalen bei : 6,04 ; 6,13 ; 6,15 ; 6,24 ; 6,27 ; 6,98 ; 7,10 ppm
IR-Spektrum (KBr, [$cm^{-1}$]) : 3 350, 1 718, 1 668, 1 720 ; 1 700, 1 520

$\Delta^{1,4}$-BNC-Methylcarbamat
Fp 180-184 °C
Elementaranalyse

| | | | |
|---|---|---|---|
| Berechnet : | C 74,36 | H 8,95 | N 3,77 |
| Gefunden : | C 74,3 | H 8,77 | N 3,68 |

$^1H$-NMR-Spektrum (80 MHz, $CDCl_3$, δ-Werte) :
18-$CH_3$ 0,78 ppm, s
19-$CH_3$ 1,23 ppm, s
21-$CH_3$ 1,15 ppm, d (J = 9 Hz)
O—$CH_3$ 3,64 ppm, s
20-CH ~ 3,64 ppm, m
— olefinische Protonen : ABC-System mit Signalen bei : 6,06 ; 6,13 ; 6,16 ; 6,26 ; 6,28 ; 6,09 ; 7,11 ppm

$\Delta^{1,4,17}$-BNC-Methylcarbamat

Elementaranalyse

| | | | |
|---|---|---|---|
| Berechnet : | C 74,76 | H 8,46 | N 3,79 |
| Gefunden : | C 74,9 | H 8,63 | N 3,90 |

$\Delta^4$-BNC-tert.-Butylcarbamat
Fp : 206-209 °C

Elementaranalyse

| | | | |
|---|---|---|---|
| Berechnet : | C 75,14 | H 9,94 | N 3,37 |
| Gefunden : | C 74,9 | H 10,08 | N 3,48 |

$^1$H-NMR-Spektrum (80 MHz, $CDCl_3$, δ-Werte) :
18-$CH_3$ 0,75 ppm, s
19-$CH_3$ 1,18 ppm, s
21-$CH_3$ 1,14 ppm, d (J = 8 Hz)
tert.-Butyl 1,43 ppm, s
20-CH ~ 3,64 ppm, m
— olefinisches Proton : 5,71 ppm, s (verbreitert).

**Ansprüche**

1. Neue 20-substituierte 3-Oxo-pregna-4-ensowie ggf. wenigstens eine weitere Doppelbindung in 1(2)- und/oder 17(20)-Stellung aufweisende Steroidverbindungen der allgemeinen Formel I

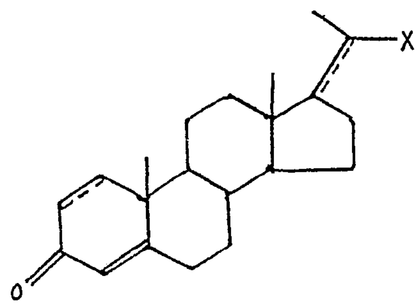

(I)

in der X die Gruppe —$CONH_2$, —NCO oder —NHCOOR bedeutet, worin R ein niederer Kohlenwasserstoffrest ist, wobei 3-Oxopregna-4-en-20-carboxamid hier nicht beansprucht wird.

2. 3-Oxo-pregna-1,4-dien-20-substituierte Steroidverbindungen der allgemeinen Formel I, in der X die Gruppe —$CONH_2$ oder —NHCOOR bedeutet.

3. 3-Oxo-pregna-1,4,17(20)-trien-20-substituierte Steroidverbindungen der allgemeinen Formel I, in der X die Gruppe —$CONH_2$ oder —NHCOOR bedeutet.

4. Verfahren zur Herstellung von 20-substituierten 3-Oxo-pregna-4-en- sowie ggf. wenigstens eine weitere Doppelbindung in 1(2)- und/oder 17(20)-Stellung aufweisenden Steroidverbindungen der allgemeinen Formel I

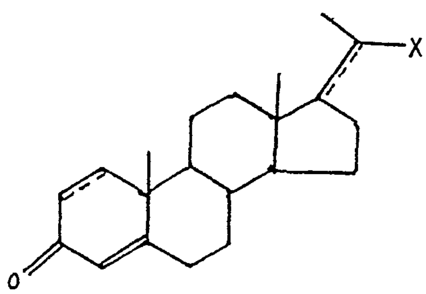

(I)

in der X die folgende Bedeutung hat : —$CONH_2$, —NCO oder —NHCOOR, worin R ein niederer Kohlenwasserstoffrest ist, wobei hier jedoch die Herstellung von 3-Oxopregna-4-en-20-carboxamid nicht beansprucht wird, dadurch gekennzeichnet, daß man von den der allgemeinen Formel I entsprechenden 20-Carbonsäuren (X = —COOH) ausgehend zunächst das 20-Carbonsäurehalogenid (X = —CO-Halogen) herstellt und dieses mit Ammoniak oder Ammoniak liefernden Verbindungen zum 20-Carbonsäureamid (X = —$CONH_2$) umsetzt, gewünschtenfalls dieses durch Umsetzung mit Bleitetraacetat in das 20-Isocyanat (X = —NCO) umlagert und weiterhin gewünschtenfalls durch Anlagerung eines Alkohols ROH (worin R die angegebene Bedeutung hat) das 20-Carbamat (X = —NHCOOR) gewinnt.

5. Verfahren nach Anspruch 4, dadurch gekennzeichnet, daß man das 20-Carbamat ohne Isolierung des 20-Isocyanats in einem Zuge aus dem 20-Säureamid gewinnt.

6. Verfahren nach Ansprüchen 4 und 5, dadurch gekennzeichnet, daß man die Umsetzungen des 20-Säureamids zum 20-Isocyanat und/oder 20-Carbamat unter Ausschluß von Wasser und vorzugsweise in inerter Atmosphäre durchführt.

## Claims

1. Novel 20-substituted 3-oxo-pregn-4-ene steroid compounds and steroid compounds having optionally at least one further double bond in the 1(2)- and/or 17(20)-positions, represented by the general formula I

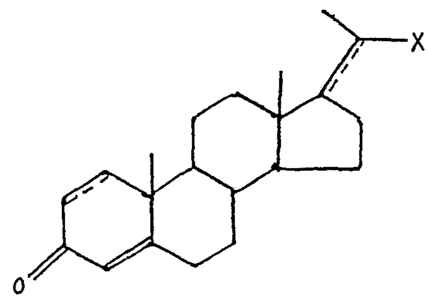

(I)

in which X denotes the group $-CONH_2$, $-NCO$ or $-NHCOOR$, wherein R is a lower hydrocarbon residue, 3-oxopregn-4-ene-20-carboxamide not being claimed here.

2. 3-oxo-pregna-1,4-diene-20-substituted steroid compounds represented by the general formula I, wherein X denotes the group $-CONH_2$ or $-NHCOOR$.

3. 3-oxo-pregna-1,4,17(20)-triene-20-substituted steroid compounds represented by the general formula I, wherein X denotes the group $-CONH_2$ or $-NHCOOR$.

4. A process for preparing 20-substituted 3-oxo-pregn-4-ene steroid compounds and steroid compounds having optionally at least one further double bond in the 1(2)- and/or 17(20)-positions, represented by the general formula I

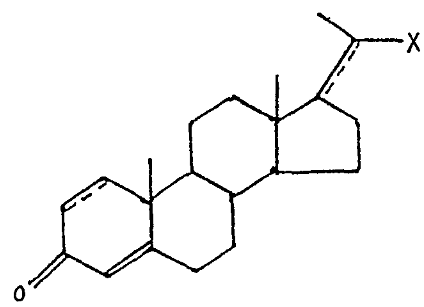

(I)

in which X has the following meaning: $-CONH_2$, $-NCO$ or $-NHCOOR$, wherein R is a lower hydrocarbon residue, 3-oxopregn-4-ene-20-carboxamide not being claimed here, characterized in that, starting from 20-carboxylic acids corresponding to the general formula I (X = $-COOH$), first the 20-carboxylic acid halide (X = $-CO$-halogen) is prepared, and this compound is reacted with ammonia or ammonia-producing compounds to give the 20-carboxylic acid amide (X = $-CONH_2$), the latter, if desired, is rearranged into the 20-isocyanate (X = $-NCO$) by reaction with lead tetraacetate, and, if further desired, the 20-carbamate (X = $-NHCOOR$) is produced by the addition of an alcohol ROH (wherein R has the meaning as defined).

5. The process according to claim 4, characterized in that the 20-carbamate is produced in one step from the 20-acid amide without recovering the 20-isocyanate.

6. The process according to claims 4 and 5, characterized in that the reactions of the 20-acid amide to give the 20-isocyanate and/or the 20-carbamate are carried out in the absence of water and preferably in an inert atmosphere.

## Revendications

1. Nouveaux stéroïdes oxo-3 prégnène-4 substitués en position 20 et présentant éventuellement au moins une autre double liaison en position 1(2) et/ou 17(20), de formule générale I

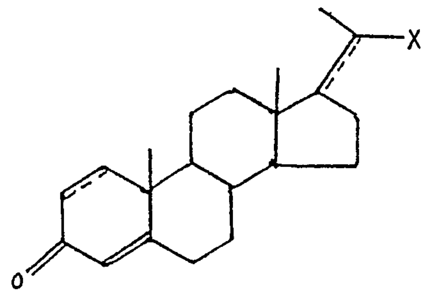

(I)

dans laquelle X représente le groupe —$CONH_2$, —NCO ou —NHCOOR, R étant un radical d'hydrocarbure inférieur et le oxo-3 prégnène-4 carboxamide n'étant pas revendiqué ici.

2. Stéroïdes oxo-3 prégnadiène-1,4 substitués en position 20 de formule générale I, dans laquelle X représente le groupe —$CONH_2$ ou —NHCOOR.

3. Stéroïdes oxo-3-prégnatriène-1,4,17(20) substitués en position 20 de formule générale I, dans laquelle X représente le groupe —$CONH_2$ ou —NHCOOR.

4. Procédé de préparation de stéroïdes oxo-3-prégnène-4 substitué en position 20 et présentant éventuellement au moins une autre double liaison en position 1(2) et/ou 17(20), de formule générale I

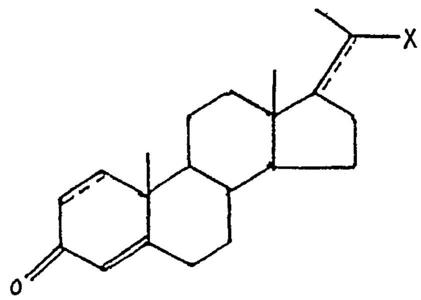

(I)

dans laquelle X a la signification suivante : —$CONH_2$, —NCO ou —NHCOOR, R étant un radical hydrocarbure inférieur et la préparation de oxo-3-prégnène-4 carboxamide n'est pas revendiquée ici, caractérisé en ce que le composé acide carboxylique correspondant à la formule générale I (X = —COOH) on prépare tout d'abord le 20-halogénure d'acide carboxylique (X = CO-halogène) et on fait réagir celui-ci avec de l'ammonique ou des composés fournissant de l'ammoniaque pour donner le 20-carboxamide (X = $CONH_2$), si on le désire on transforme celui-ci en 20-isocyanate (X = NCO) par réaction avec du tétraacétate de plomb et en outre si on le désire, par suppression d'un alcool ROH (R ayant la signification indiquée) on obtient le 20-carbamate (X = —NHCOOR).

5. Procédé selon la revendication 4, caractérisé en ce que l'on obtient le 20-carbamate directement en une seule opération à partir du 20-amide sans isoler le 20-isocyanate.

6. Procédé selon la revendication 4 et 5, caractérisé en ce que l'on réalise les transformations du 20-amide en 20-isocyanate et/ou en 20-carbamate en l'absence totale d'eau et de préférence en atmosphère inerte.